# EUROPEAN PATENT APPLICATION

(11) **EP 2 090 584 A1**
(43) Date of publication of application: **19.08.2009**
(21) Application number: 08002685.9
(22) Date of filing: 13.02.2008
(51) Int. Cl.: C07K 14/46

(54) **Identification of a novel cysteine-rich cell penetrating peptide**

(71) Applicant: Max-Planck-Gesellschaft zur Förderung der Wissenschaften e.V., 80539 München (DE)
(72) Inventor: Ugurbil, Kamil, 72076 Tübingen (DE); Jha, Deepti, 72076 Tübingen (DE); Engelmann, Jörn, 72076 Tübingen (DE); Mishra, Ritu, 72076 Tübingen (DE); Wiesmüller, Karl-Heinz, 72070 Tübingen (DE)
(74) Representative: Vossius & Partner

(57) **Abstract**

The present invention relates to a nucleic acid molecule encoding a peptide capable of being internalized into a cell, wherein said nucleic acid molecule comprises (a) a nucleic acid molecule encoding a peptide having the amino acid sequence of SEQ ID NO: 2; (b) a nucleic acid molecule having the DNA sequence of SEQ ID NO: 1, wherein T is U if the nucleic acid molecule is RNA; (c) a nucleic acid molecule hybridizing under stringent conditions to the complementary strand of a nucleic acid molecule of (a) or (b), wherein the peptide encoded by said nucleic acid molecule has a cysteine at least at two positions selected from the group consisting of positions 1, 7 and 8 of SEQ ID NO: 2 and an arginine or a lysine at least at four positions selected from the groups consisting of position 2, 4, 6, 9 or 10 of SEQ ID NO: 2; (d) a nucleic acid molecule encoding a peptide having at least 70% sequence identity with that of SEQ ID NO: 2, wherein at least at two positions selected from the group consisting of positions 1, 7 and 8 of SEQ ID NO: 2 a cysteine is present and wherein at least at four positions selected from the groups consisting of position 2, 4, 6, 9 or 10 of SEQ ID NO: 2 an arginine or a lysine is present; or (e) a nucleic acid molecule degenerate with respect to the nucleic acid molecule of (c) or (d). The present invention also relates to a peptide encoded by the nucleic acid of the invention, a fusion molecule comprising the peptide of the invention and a composition comprising the peptide or the fusion molecule of the invention. Furthermore, the present invention relates to a method of detecting the internalization behaviour of a fusion molecule of the invention, the composition of the invention for treating and/or preventing a condition selected from cancer, enzyme deficiency diseases, infarcts, cerebral ischemia, diabetes, inflammatory diseases, infections such as bacterial, viral or fungal infections, autoimmune diseases such as systemic lupus erythematodes (SLE) or rheumatoid arthritis, diseases with amyloid-like fibrils such as Alzheimer's disease (AD) and Parkinson's disease (PD) or certain forms of myopathy.

SEQUENCE LISTING

<110> Max-Planck-Gesellschaft zur Förderung der Wissenschaften
e.V.
EMC microcollections GmbH

<120> Identification of a novel cysteine-rich cell penetrating peptide

<130> N2735 EP

<160> 2

<170> PatentIn version 3.3

<210> 1
<211> 30
<212> DNA
<213> artificial sequence

<220>
<221> source
<223> /note="Description of artificial sequence: DNA encoding the optimized CPP
sequence"

<400> 1
tgtcgttggc gttggaaatg ttgtaaaaaa 30

<210> 2
<211> 10
<212> PRT
<213> artificial sequence

<220>
<221> source
<223> /note="Description of artificial sequence: optimized CPP sequence"

<220>
<221> variation
<222> 3
<223> /replace="c"

<220>
<221> variation
<222> 6
<223> /replace="c"
/replace="a"
/replace="g"

<220>
<221> variation
<222> 12
<223> /replace="c"
/replace="a"
/replace="g"

<220>
<221> variation
<222> 18
<223> /replace="g"

<220>
<221> variation
<222> 21

<223>  /replace="c"

<220>
<221>  variation
<222>  24
<223>  /replace="c"

<220>
<221>  variation
<222>  27
<223>  /replace="g"

<220>
<221>  variation
<222>  30
<223>  /replace="g"

<400>  2

Cys Arg Trp Arg Trp Lys Cys Cys Lys Lys
1               5                   10

## Description

The present invention relates to a nucleic acid molecule encoding a peptide capable of being internalized into a cell, wherein said nucleic acid molecule comprises (a) a nucleic acid molecule encoding a peptide having the amino acid sequence of SEQ ID NO: 2; (b) a nucleic acid molecule having the DNA sequence of SEQ ID NO: 1, wherein T is U if the nucleic acid molecule is RNA; (c) a nucleic acid molecule hybridizing under stringent conditions to the complementary strand of a nucleic acid molecule of (a) or (b), wherein the peptide encoded by said nucleic acid molecule has a cysteine at least at two positions selected from the group consisting of positions 1, 7 and 8 of SEQ ID NO: 2 and an arginine or a lysine at least at four positions selected from the group consisting of positions 2, 4, 6, 9 or 10 of SEQ ID NO: 2; (d) a nucleic acid molecule encoding a peptide having at least 70% sequence identity with that of SEQ ID NO: 2, wherein at least at two positions selected from the group consisting of positions 1, 7 and 8 of SEQ ID NO: 2 a cysteine is present and wherein at least at four positions selected from the group consisting of positions 2, 4, 6, 9 or 10 of SEQ ID NO: 2 an arginine or a lysine is present; or (e) a nucleic acid molecule degenerate with respect to the nucleic acid molecule of (c) or (d). The present invention also relates to a peptide encoded by the nucleic acid of the invention, a fusion molecule comprising the peptide of the invention and a composition comprising the peptide or the fusion molecule of the invention. Furthermore, the present invention relates to a method of detecting the internalization behaviour of a fusion molecule of the invention, the composition of the invention for treating and/or preventing a condition selected from cancer, enzyme deficiency diseases, infarcts, cerebral ischemia, diabetes, inflammatory diseases, infections such as bacterial, viral or fungal infections, autoimmune diseases such as systemic lupus erythematodes (SLE) or rheumatoid arthritis, diseases with amyloid-like fibrils such as Alzheimer's disease (AD) and Parkinson's disease (PD) or certain forms of myopathy.

In this specification, a number of documents are cited. The disclosure content of these documents including manufacturers' manuals is herewith incorporated by reference in its entirety.
The targeted delivery of substances to cells has long been hampered by the cell membrane being an efficient protective wall to exclude most molecules that are not actively imported by living cells. Only a narrow range of molecules of certain molecular weight, polarity and net charge is able to diffuse through cell membranes. Other molecules have to be actively transported by e.g. receptor-mediated endocytosis or artificially forced through the cell membrane by methods such as electroporation, cationic lipids/liposomes, micro-injection, viral delivery, encapsulation in polymers . These methods are mainly utilized to deliver hydrophobic molecules. Furthermore, the side effects associated with these methods and the fact that their utilization is limited to in vitro uses has prevented them from becoming an efficient means to deliver substances such as drugs to the cell in order to treat diseases and conditions.
The discovery of cell-penetrating peptides (CPPs) also called protein transduction domains (PTDs) or membrane translocation sequences (MTS) proved that the translocation of larger molecules through the cell membrane is possible. Prominent examples of CPPs are the HIV-1 TAT translocation domain (Green and Loewenstein, 1988) and the homeodomain of the Antennapedia protein from Drosophila (Joliot et al., 1991). The exact translocation mechanism is still disputed. Mutation studies of the Antennapedia protein revealed that a sequence of 16 amino acids called penetratin or pAntp (Derossi et al., 1994) is necessary and sufficient for membrane translocation. In the following, other protein-derived CPPs were developed such as the basic sequence of the HIV-1 Tat protein (Vivès et al., 1997) and the chimeric peptide transportan (Pooga et al., 1998). A synthetic peptide developed is the amphipathic model peptide (Oehlke et al., 1998). Coupling of antisense DNA or PNAs to CPPs was shown to exert the desired effect in vivo.
It was long questioned which features were necessary for a CPP to exert the translocation function. In general, little structural resemblance has been found between the different families of CPPs. So far the only consistently found feature is the high content of basic amino acids resulting in a positive net charge. Thus, it is assumed that CPPs initially bind to negatively charged head groups of lipids or proteins in the cell membrane. In this regard, the importance of arginine as positive amino acid was demonstrated by several groups (Rothbard et al., 2000; Wender et al., 2000). Generally, an alpha-helical secondary structure has been predicted for CPPs which could be verified for some cases but cannot be taken as a general prerequisite.
Many proteins able to translocate have severe side-effects on the cell, which is understandable in view of the fact that most of the naturally occurring substances are used as e.g. antimicrobial substances or toxins. CPPs can e.g. cause cytoplasmic leakage due to membrane disruption and also interfere with the functioning of membrane proteins. CPPs might also exhibit cellular toxic effects, such as e.g. transportan which affects GTPase activity (Soomets et al., 2000). Furthermore, it becomes more and more clear that many CPPs only exert their function under certain very narrow conditions which cannot be met in vivo. Another drawback is that, depending on the target cell, the CPPs may be rapidly degraded in the cells. Lastly, toxic and immunogenic effects of CPPs have been observed which prevent their utilization e.g. in therapeutic applications.

Crotamine is one of the main toxins in the venom of the South American rattlesnake (Rádis-Baptista et al., 1999) and shows high homology with other venom myotoxins. The 42 amino acid long cationic polypeptide contains 11 basic residues and six cysteines giving rise to three disulfide bonds. It has two putative NLS motifs, Crot₂₋₁₈ and Crot₂₇₋₃₉. Crotamine was shown to be a CPP penetrating into different cell types and mouse blastocysts in vitro (Kerkis et al., 2004). It was shown to be non-toxic to a concentration of up to 1 µM and to localize preferably in the nucleus where it is supposed to bind to chromatin structures. When applied before cell division, crotamine is mainly localized in the cytoplasm after the telophase. Up to now and depending on the mechanism of internalization, known CPPs mainly localize in the nucleus or, in case they are internalized in vesicles, remain there and only a small part is released into the cytoplasm.

Thus, there is a need for CPPs with improved properties.

Accordingly, the present invention relates to a nucleic acid molecule encoding a peptide capable of being internalized into a cell, wherein said nucleic acid molecule comprises (a) a nucleic acid molecule encoding a peptide having the amino acid sequence of SEQ ID NO: 2; (b) a nucleic acid molecule having the DNA sequence of SEQ ID NO: 1, wherein T is U if the nucleic acid molecule is RNA; (c) a nucleic acid molecule hybridizing under stringent conditions to the complementary strand of a nucleic acid molecule of (a) or (b), wherein the peptide encoded by said nucleic acid molecule has a cysteine at least at two positions selected from the group consisting of positions 1, 7 and 8 of SEQ ID NO: 2 and an arginine or a lysine at least at four positions selected from the group consisting of positions 2, 4, 6, 9 or 10 of SEQ ID NO: 2; (d) a nucleic acid molecule encoding a peptide having at least 70% sequence identity with that of SEQ ID NO: 2, wherein at least at two positions selected from the group consisting of positions 1, 7 and 8 of SEQ ID NO: 2 a cysteine is present and wherein at least at four positions selected from the group consisting of positions 2, 4, 6, 9 or 10 of SEQ ID NO: 2 an arginine or a lysine is present; or (e) a nucleic acid molecule degenerate with respect to the nucleic acid molecule of (c) or (d).

The term "nucleic acid molecule" as used interchangeably with the term "polynucleotide", in accordance with the present invention, includes DNA, such as cDNA or genomic DNA, and RNA. If the nucleic acid molecule is RNA, thymine (T) bases denoted in e.g. SEQ ID NO: 1 are replaced with uracil (U), the thymine analogue occurring in RNA. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. They may contain additional non-natural or derivative nucleotide bases, as will be readily appreciated by those skilled in the art. For the purposes of the present invention, also a peptide nucleic acid (PNA) can be applied. Peptide nucleic acids have a backbone composed of repeating N-(2-aminoethyl)-glycine units linked by peptide bonds. The purine and pyrimidine bases are linked to the backbone by methylene carbonyl bonds.
In a preferred embodiment, the nucleic acid molecule is DNA.

The term "peptide" as used herein describes linear molecular chains of amino acids, including fragments of single chain proteins, containing up to 30 amino acids. Peptides may form oligomers consisting of at least two identical or different molecules. The corresponding higher order structures of such multimers are, correspondingly, termed homo- or heterodimers, homo- or heterotrimers etc.. The term "peptide" furthermore comprises peptidomimetics of such peptides where amino acid(s) and/or peptide bond(s) have been replaced by functional analogues. Such functional analogues also include all known amino acids other than the 20 gene-encoded amino acids, such as selenocysteine.
A polypeptide as used in the context of the present invention contains more than 30 amino acids. In accordance with the invention, the term is interchangeably used with "protein".
The terms "peptide" and "polypeptide" also refer to naturally modified peptides and polypeptides where the modification is effected e.g. by glycosylation, acetylation, phosphorylation and similar modifications which are well known in the art.

The term "capable of being internalized" as used in the context of the present invention refers to the ability of some peptides to pass the plasma membrane of cells. Different mechanisms of internalization are proposed in the literature: an energy-dependent endocytotic mechanism and an energy-independent passive transport mechanism. The latter can be further divided into several suggested models. In the inverted micelle-driven delivery model, the positively charged part of the CPP interacts with the phospholipids in the membrane, followed by the interaction of the hydrophobic part of the peptide with the membrane, creating the inverted micelle. Another model suggests direct penetration of the plasma membrane. It was suggested by example of the TAT peptide that the mechanism of translocation depends on the cargo attached to the peptide. Size may play a role as well as the chemical properties of the cargo. Furthermore, it was shown that the mechanisms may vary depending on the concentration of CPP. For a recent review see e.g. Tréhin and Merkle (2004), Magzoub and Gräslund (2004) or Gupta et al. (2005). In the context of the present invention, any possible mechanism of internalization is envisaged. A preferred mechanism would ensure that at least a part, preferably more than 30%, more preferably more than 40%, even more preferably more than 50%, even more preferably more than 60%, even more preferably more than 70% and most preferably more than 80% of the CPP conjugate localizes in the cytoplasm in contrast to localization in different compartments, e.g. in vesicles, endosomes or in the nucleus.

The term "hybridizes/hybridizing" as used herein refers to a pairing of a nucleic acid molecule to a (partially) complementary strand of this nucleic acid molecule which thereby forms a hybrid.

It is well known in the art how to perform hybridization experiments with nucleic acid molecules. Correspondingly, the person skilled in the art knows what hybridization conditions she/he has to use to allow for a successful hybridization in accordance with item (c), above. The establishment of suitable hybridization conditions is referred to in standard text books such as Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (2002), or Higgins and Hames (Eds.) "Nucleic acid hybridization, a practical approach" IRL Press Oxford, Washington DC, (1985).

"Stringent conditions" refers to hybridization conditions under which the polynucleotides that are capable of hybridizing to the polynucleotides of the invention or parts thereof do not cross hybridize to unrelated polynucleotides. Appropriate stringent hybridization conditions for each nucleic acid sequence may be established by a person skilled in the art on well-known parameters such as temperature, composition of the nucleic acid molecules (GC-content), salt conditions etc.; see, for example, Sambrook et al., "Molecular Cloning, A Laboratory Manual"; CSH Press, Cold Spring Harbor, 1989 or Higgins and Hames (eds.), loc. cit., see in particular the chapter "Hybridization Strategy" by Britten & Davidson, 3 to 15. Such conditions comprise e.g. an overnight incubation at 65°C in 4x SSC (600 mM NaCl, 60 mM sodium citrate) followed by washing at 65°C in 0.1x SSC for one hour. Alternatively, hybridization conditions can comprise: an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing in e.g. 0.1-0.5x SSC at about 55-65°C for about 5 to 20 min. In general, for achieving "high stringency", salt concentrations of 0.0165 to 0.033 M and a temperature of 5 to 10 °C below the melting temperature (Tm) of the nucleic acid are sufficient. Only sequences with a high degree of identity will hybridize under these conditions.
It is of note that variations in the above conditions may be accomplished through the inclusion and/or substitution of alternate blocking reagents. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Changes in the stringency of hybridization are primarily accomplished through the manipulation of formamide concentration (lower percentages of formamide result in lowered stringency), salt conditions (lower salt concentrations result in lowered stringency), or temperature. Such modifications can generally be effected by the skilled person without further ado.
As disclosed by Nielsen et al., Science 254:1497 (1991); and Egholm et al., Nature 365:666 (1993), PNAs bind specifically and tightly to complementary DNA strands and are not degraded by nucleases. In fact, PNA binds more strongly to DNA than DNA itself does. This is probably because there is no electrostatic repulsion between the two strands, and also the polyamide backbone is more flexible. Because of this, PNA/DNA duplexes bind under a wider range of stringency conditions than DNA/DNA duplexes, making it easier to perform multiplex hybridization. Smaller probes can be used than with DNA due to the strong binding. In addition, it is more likely that single base mismatches can be determined with PNA/DNA hybridization because a single mismatch in a PNA/DNA 15-mer lowers the melting point (T.sub.m) by 8°-20° C, vs. 4°-16° C for the DNA/DNA 15-mer duplex. Also, the absence of charge groups in PNA means that hybridization can be done at low ionic strengths and reduce possible interference by salt during the analysis.

A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed).
Examples for hybridization assays comprise without limitation Northern and Southern blot assays, heteroduplex analysis, detection of mutations by sequence specific oligonucleotide hybridization, allele-specific oligonucleotide hybridization on DNA chips, assays based on the Illumina's^{®} technology, assays based on the BeadArray^{®} technology, see, for example, Barnes et al., Nucleic Acids Res. 33 (2005) 5914-5923; Fan et al., Biotechniques 39 (2005) 583-588; Shen et al., Mutat. Res. 573 (2005) 70-82; Steemers and Gunderson, Pharmacogenomics, 6 (2005) 777-782.

Nucleic acid molecules are "complementary" if they naturally bind to each other under permissive salt and temperature conditions by base-pairing. For example, the sequence "A-G-T" binds to the complementary sequence "T-C-A". "Complementary" in accordance with the invention, refers to the complete base pairing of nucleotides over the entire length of the nucleic acid molecule of the invention.

As regards the presence of specific amino acids at certain positions of the peptide encoded by the nucleic acid molecule of the present invention, these positions can also be assigned to the sequence of said peptide if it is present in a longer peptide or protein. More particularly, if the stretch of amino acids homologous or identical to the peptide encoded by SEQ ID NO: 1 is identified in a nucleic acid sequence encoding a longer peptide or protein, both sequences can be aligned and the positions are assigned. From this information, the positions in the longer peptide or protein corresponding to the respective amino acid in the peptide encoded by SEQ ID NO: 1 can be retrieved.

In accordance with the present invention, the term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g., 70%, 80% or 85% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of a test sequence.
To evaluate the identity level between two nucleotide or protein sequences, they can be aligned electronically using suitable computer programs known in the art. Such programs comprise BLAST (Altschul et al., J. Mol. Biol. 1990, 215: 403), variants thereof such as WU-BLAST (Altschul & Gish, Methods Enzymol. 1996, 266: 460), FASTA (Pearson & Lipman, Proc. Natl. Acad. Sci. USA 1988, 85: 2444) or implementations of the Smith-Waterman algorithm (SSEARCH, Smith & Waterman, J. Mol. Biol. 1981, 147: 195). These programs, in addition to providing a pairwise sequence alignment, also report the sequence identity level (usually in percent identity) and the probability for the occurrence of the alignment by chance (P-value). The BLASTN program for nucleic acid sequences uses as default a word length (W) of 11, an expectation (E) of 10, M=5, N=4, and a comparison of both strands. For amino acid sequences, the BLASTP program uses as default a word length (W) of 3, and an expectation (E) of 10. The BLOSUM62 scoring matrix (Henikoff, Proc. Natl. Acad. Sci., 1992, 89:10915) uses alignments (B) of 50, expectation (E) of 10, M=5, N=4, and a comparison of both strands. Programs such as CLUSTALW (Thompson Nucl. Acids Res. 2 (1994), 4673-4680) can be used to align more than two sequences. In addition, CLUSTALW, unlike e.g. FASTDB, does take sequence gaps into account in its identity calculations.
All of the above programs can be used in accordance with the invention.
In a preferred embodiment of the present invention, the sequence identity to SEQ ID NO: 2 is at least 80%, more preferably at least 90% and most preferred at least 95%.

Substitutions in the amino acid sequence of the peptide of the present invention are preferably conservative. This means that substitutions preferably take place within one class of amino acids. For example, a positively charged amino acid is preferably mutated to another positively charged amino acid. The same holds true for the classes of basic, aromatic or aliphatic amino acids.

Degenerate in accordance with the present invention refers to the degeneracy of the genetic code. Degeneracy results because a triplet code designates 20 amino acids and a stop codon. Since four bases exist which are utilized to encode genetic information, triplet codons are required to produce at least 21 different codes, i.e. one for each of the 20 gene-encoded amino acids and one for a stop codon. The possible 4³ possibilities for bases in triplets give 64 possible codons, meaning that some degeneracy must exist. As a result, some amino acids are encoded by more than one triplet, i.e. by up to six. The degeneracy mostly arises from alterations in the third position in a triplet. Accordingly, nucleic acid molecules having a different sequence than that specified above, but still encoding the same peptide lie within the scope of the present invention.

In the course of the present invention, it has surprisingly been found that a peptide derived from crotamine but having a reduced length as compared to a fragment of crotamine consisting of amino acids 27 to 39 (Cro₂₇₋₃₉₎ and retaining all cysteines has improved internalization properties as compared to naturally occurring crotamine or Cro₂₇₋₃₉. The C-terminal nuclear localization signal Cro₂₇₋₃₉ (KMDCRWRWKCCKK) proposed as one of two potential sequences responsible for membrane permeation (Kerkis et al., 2004) was separately examined for its internalization properties. The uptake behavior of this fragment was studied with a fluorophore attached to it. It proved to be an efficient CPP also showing cytoplasmic diffusion. Cytoplasmic diffusion is particularly desirable to deliver pharmacologically active substances to the cell. This additional feature led the present inventors to introduce changes to this fragment to avoid the use of amino acids like methionine, tryptophan, aspartic acid and, in particular, cysteine which makes the synthesis more complex and challenging. Avoiding cysteines will not only facilitate the synthesis, handling and storage of the peptide but is also suggested to improve the in vivo properties of the peptide since cysteines will likely form intra- and intermolecular cysteine bridges and therefore promote aggregation of the peptide. The absence or presence of disulphide bonds can be important for peptides to maintain their biological activity and conformational stability. However, up to now, the role of cysteines or cysteine bridges in CPP has not been examined. International patent application WO03/106491 discloses a method for predicting or designing CPPs. It is of note that the majority of peptides predicted and/or shown to exert CPP properties do not contain any cysteine.
Positively charged amino acids (lysine and arginines) were not modified as these are an important feature of a cell penetrating peptide. Different derivatives wherein cysteines were substituted with alpha amino butyric acid or serine (close analogues of cysteine) were synthesized as well as fragments of the sequence Cro₂₇₋₃₉ by deleting amino acids from the N-terminus. Alternatively, cysteines were deleted one by one or amino acids anywhere in the sequence were deleted. Finally, tryptophan was substituted by proline or phenylalanine. All combinations were synthesized containing a lysine at the N-terminus as a linker for the coupling of FITC (at the ε-amino group) for analyzing samples for intracellular uptake by fluorescence imaging.

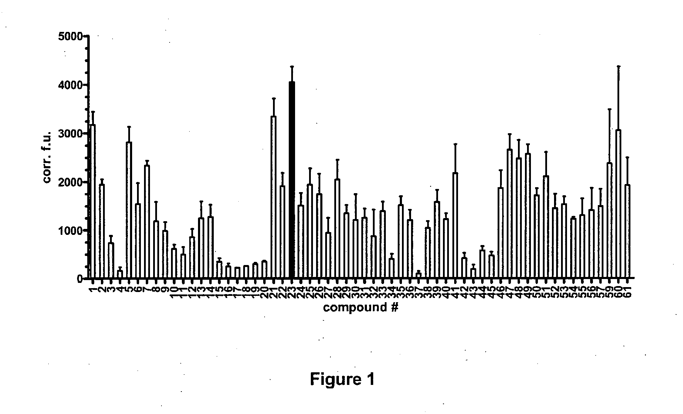

The internalization studies on cultured cells show that a change in the amino acid composition dramatically affects the intracellular uptake (see Figures 1 to 3 and the examples). Not only the deletion of cysteines one by one in different combinations but also the substitution by α-aminobutyric acid reduced the cellular uptake with the number of cysteines deleted or substituted. Substitution of cysteines by serine shows nearly the same results. Since proline-rich peptides are known to enhance cell permeability, tryptophan was substituted with proline. However, internalization was again immensely affected to the negative. The best results are observed for a fragment K(FITC)-CRWRWKCCKK (peptide 23 of table 1 below displaying a complete list of all sequences examined for their internalization properties) which is competently taken up by cells and also show endosomal as well as cytoplasmic fluorescence distribution. This fragment without the linker is three amino acids shorter than the original fragment Cro₂₇₋₃₉ having the sequence KMDCRWRWKCCKK) keeping the number of the cysteines the same. Studies carried out on well known CPPs like Tat, Antennapedia and polyarginines revealed that the role of the positive charge is crucial for translocation. Unlike the known CPPs the CPP of the present invention is markedly different in terms of its function. Efficient cellular uptake and cytosolic location along with vesicular distribution at subtoxic concentrations (<2.5 µM) is the distinguishing feature which is also shown by other CPP but at comparatively high concentrations (>10 µM). Other distinctive features are the influence of the chirality of the peptide backbone as well as the sequence order on the cellular uptake: Forms of the proposed peptide with the sequence of the CPP of the present invention reversed, the sequence of the CPP of the present invention with D-amino acids, or with D-amino acids and in reversed order also showed lower uptake and cytosolic diffusion unlike known for the Tat peptide.
The entire study shows the significance of each amino acid focusing the requirement of charge and hydrophobic residues during membrane permeation. As known from previous studies charged residues help to adhere to the cell surface which is the first step of internalization and then tryptophan might aid membrane translocation by membrane destabilization.

Thus, it has been found that the CPP of the invention actually requires tryptophanes and, surprisingly, cysteines in order to optimally exert its internalization properties. Accordingly, an optimized peptide according to the present invention is encoded by the nucleic acid sequence depicted in SEQ ID NO: 1 or has the amino acid sequence of SEQ ID NO: 2. It encodes three cysteines and two tryptophanes which unexpectedly are shown to improve the internalization properties of the peptide.
Being rich in amino acids like methionine, aspartic acid, and cysteine Cro₂₇₋₃₉ endures difficulties during synthesis and storage. Thus it becomes necessary to avoid such amino acids by replacement or deletion as much as possible which is the main goal of this study. Herein we showed the development of the proposed fragment from the known one. Both the sequences are comparable in terms of function (internalization efficiency and cytosolic distribution) but the proposed sequence seems to give the same results being smaller in length and avoiding at least methionine and aspartic acid.

**Table 1: Sequences derived from Cro₂₇₋₃₉ and examined for their internalization properties in the present invention. The individual sequences are discussed in further detail in the appended examples. X: α-aminobutyric acid; Capitals denote L-amino acids, minuscules denote D-amino acids**

| | |
|---|---|
| 1 | K (FITC) - KMDCRWRWKCCKK (Cro₂₇₋₃₉) |
| 2 | K (FITC) - KMDXRWRWKCCKK |
| 3 | K (FITC) - KMDXRWRWKXCKK |
| 4 | K (FITC) - KMDXRWRWKXXKK |
| 5 | K (FITC) - KMDXRWRWKCXKK |
| 6 | K (FITC) - KMDCRWRWKCXKK |
| 7 | K (FITC) - KMDCRWRWKCSKK |
| 8 | K (FITC) - KMDCRWRWKSCKK |
| 9 | K (FITC) - KMDSRWRWKCCKK |
| 10 | K (FITC) - KMDCRWRWKSSKK |
| 11 | K (FITC) - KMDSRWRWKSSKK |
| 12 | K (FITC) - KMDSRWRWKSCKK |
| 13 | K (FITC) - KMDSRWRWKCSKK |
| 14 | K (FITC) - KMDCRWRPKCCKK |
| 15 | K (FITC) - KMDCRPRPKCCKK |
| 16 | K (FITC) - KMDXRPRPKCCKK |
| 17 | K (FITC) - KMDXRPRPKXCKK |
| 18 | K (FITC) - KMDXRPRPKCXKK |
| 19 | K (FITC) - KMDCRPRPKXCKK |
| 20 | K (FITC) - KMDCRPRPKCXKK |
| 21 | K (FITC) - MDCRWRWKCCKK |
| 22 | K (FITC) - DCRWRWKCCKK |
| 23 | K (FITC) - **CRWRWKCCKK** |
| 24 | K (FITC) - RWRWKCCKK |
| 25 | K (FITC) - MDCRWRWKXCKK |
| 26 | K (FITC) - DCRWRWKXCKK |
| 27 | K (FITC) - DCRWRWKCXKK |
| 28 | K (FITC) - CRWRWKXCKK |
| 29 | K (FITC) - CRWRWKCXKK |
| 30 | K (FITC) - RWRWKXCKK |
| 31 | K (FITC) - MDCRWRWKXXKK |
| 32 | K (FITC) - DCRWRWKXXKK |
| 33 | K (FITC) - CRWRWKXXKK |
| 34 | K (FITC) - RWRWKXXKK |
| 35 | K (FITC) - KMDCRWRWKCKK |
| 36 | K (FITC) - KMDCRWRWKKK |
| 37 | K (FITC) - KMDRWRWKKK |
| 38 | K (FITC) - KDCRWRWKCCKK |
| 39 | K (FITC) - KCRWRWKCCKK |
| 40 | K (FITC) - KRWRWKCCKK |
| 41 | K (FITC) - CRWRWKCSKK |
| 42 | K (FITC) - CRWRWKSSKK |
| 43 | K (FITC) - SRWRWKSSKK |
| 44 | K (FITC) - SRWRWKCSKK |
| 45 | K (FITC) - SRWRWKSCKK |
| 46 | K (FITC) - SRWRWKCCKK |
| 47 | K (FITC) - CRFRWKCCKK |
| 48 | K (FITC) - CRWRFKCCKK |
| 49 | K (FITC) - CRFRFKCCKK |
| 50 | K (FITC) - KCCKWRWRCK |
| 51 | k (FITC) - crwrwkcckk |
| 52 | k (FITC) - kcckwrwrck |
| 53 | K (FITC) - CrWRWKCCKK |
| 54 | K (FITC) - CRwRWKCCKK |
| 55 | K (FITC) - CRWrWKCCKK |
| 56 | K (FITC) - CRWRwKCCKK |
| 57 | K (FITC) - CrwrwKCCKK |
| 58 | K (FITC) - CRWRWKCGCKK |
| 59 | K (FITC) - CCRWRWKCCKK |
| 60 | K (FITC) - CGCRWRWKCGCKK |
| 61 | K (FITC) - CRWRWKCG |

In a preferred embodiment, the peptide encoded by the nucleic acid molecule further comprises a linker.

A linker as used in connection with the present invention is used to connect the peptide of the invention with other moieties. The linker serves to physically separate the peptide of the invention and the other moiety or moieties and to ensure that neither the peptide of the invention nor the other moieties are limited in their function due to the close vicinity to the other. Depending on the other moiety, the linker can be a peptide bond, an amino acid, a peptide of appropriate length, or a different molecule providing the desired features. The skilled person knows how to design appropriate linker molecules, in particular linker peptides based on his common knowledge. For example, peptide linkers can be chosen from the LIP (Loops in Proteins) database (Michalsky et al., 2003). A linker may be appended to the N- or the C-terminus or, if deemed suitable, also to an amino acid apart from the terminal amino acids of the peptide of the present invention. The linker is preferably located at the N-terminus.
A moiety as used in connection with the present invention is a functional unit. A moiety can e.g. be a linker minimally comprising a lysine. Other moieties can be e.g. selectable markers such as FITC or drugs as described in more detail below.

In a more preferred embodiment, the linker is a lysine. The ε-amino group in lysine is suitable to couple the peptide of the invention to various other moieties. Furthermore, the lysine may serve to further improve the internalization properties of the peptide of the invention and may then be supplemented by another linker if deemed suitable.

The present invention also relates to a vector comprising the nucleic acid molecule of the present invention. Preferably, the vector is a plasmid, cosmid, virus, bacteriophage or another vector used conventionally e.g. in genetic engineering. An expression vector according to this invention is capable of directing the replication and the expression of the nucleic acid molecule of the invention and the peptide or polypeptide encoded thereby. Generally, vectors can contain one or more origins of replication (ori) and inheritance systems for cloning or expression, one or more markers for selection in the host and one or more expression cassettes. Methods which are well known to those skilled in the art can be used to construct and modify recombinant vectors; see, for example, the techniques described in Sambrook and Russell, 2001 and Ausubel, 2001.

The coding sequences inserted in the vector can e.g. be synthesized by standard methods, or isolated from recombinant sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e.g., translation initiation codon, promoters, enhancers, and/or insulators), internal ribosomal entry sites (IRES) (Owens *et al*., 2001) and optionally poly-A signals ensuring termination of transcription and stabilization of the transcript. Additional regulatory elements may include transcriptional as well as translational enhancers, and/or naturally-associated or heterologous promoter regions. Preferably, the nucleic acid molecule of the invention is operably linked to such expression control sequences allowing expression in prokaryotes or eukaryotic cells e.g. in the form of a vector. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the nucleic acid molecule of the invention. Such leader sequences are well known in the art. Specifically-designed vectors allow the shuttling of DNA between different hosts, such as between bacteria and fungal cells or bacteria and animal cells.

The nucleic acid molecules of the invention as described herein above may be designed for direct introduction or for introduction via liposomes, phage vectors or viral vectors (e.g. adenoviral, retroviral) into the cell. Additionally, baculoviral systems or systems based on Vaccinia Virus or Semliki Forest Virus can be used as vector in eukaryotic expression system for the nucleic acid molecules of the invention. Other expression vectors derived from viruses and usable for delivery of the polynucleotides or vector into targeted cell populations are retroviruses, vaccinia virus, adeno-associated virus, herpes viruses, or bovine papilloma virus.

A typical origin of replication in mammalian vectors is the SV40 viral ori. Additional elements in mammalian vectors might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Other examples for regulatory elements permitting expression in eukaryotic host cells are the *AOX1* or *GAL1* promoter in yeast or the CMV- (Cytomegalovirus), SV40-, RSV-promoter (Rous sarcoma virus), chicken beta-actin promoter, CAG-promoter (a combination of chicken beta-actin promoter and cytomegalovirus immediate-early enhancer), the gai10 promoter, human elongation factor 1α-promoter, CMV enhancer, CaM-kinase promoter, the Autographa californica multiple nuclear polyhedrosis virus (AcMNPV) polyhedral promoter or a globin intron in mammalian and other animal cells. Besides elements which are responsible for the initiation of transcription such regulatory elements may also comprise transcription termination signals, such as the SV40-poly-A site or the tk-poly-A site or the SV40, IacZ and AcMNPV polyhedral polyadenylation signals, downstream of the polynucleotide. Suitable selectable markers are dhfr, gpt, G418 neomycin, hygromycin allows the identification and isolation of the transfected cells. The transfected nucleic acid can also be amplified to express large amounts of the encoded peptide. The dhfr (dihydrofolate reductase) marker is useful to develop cell lines that carry several hundred or even several thousand copies of the gene of interest. Another useful selection marker is the enzyme glutamine synthase (GS) (Murphy et al. 1991; Bebbington et al. 1992). Using these markers, the mammalian cells are grown in selective medium and the cells with the highest resistance are selected.

Suitable origins of replication for prokaryotic cells include, for example, the Col E1 and the M 13 origins of replication. Examples of suitable markers for culturing in *E. coli* and other prokaryotes include tetracycline, kanamycin or ampicillin resistance genes. Possible regulatory elements permitting expression in prokaryotic host cells comprise, e.g., the lac, *trp* or *tac* promoter, the lacUV5 or the trp promotor in *E. coli*.

The nucleic acid molecule of the present invention may be inserted into several commercially available vectors well known to the skilled person who is also able to determine which vectors are suitable for the introduction and/or expression of the peptide of the invention. Non-limiting examples include prokaryotic plasmid vectors, such as the pUC-series, pBluescript (Stratagene), the pET-series of expression vectors (Novagen) or pCRTOPO (Invitrogen), lambda gt11, pJOE, the pBBR1-MCS series, pJB861, pBSMuL, pBC2, pUCPKS, pTACT1 and vectors compatible with expression in mammalian cells like pREP (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, Okayama-Berg cDNA expression vector pcDV1 (Pharmacia), pRc/CMV, pcDNA1, pcDNA3 (Invitrogene), pSPORT1 (GIBCO BRL), pGEMHE (Promega), pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pAO815, pPIC9K and pPIC3.5K (all Intvitrogen).

The nucleic acid molecule of the present invention referred to above may also be inserted into vectors such that a translational fusion with another nucleic acid molecule is generated. The other nucleic acid molecule may encode a protein which may e.g. increase the solubility and/or facilitate the purification of the peptide encoded by the nucleic acid molecule of the invention. Non-limiting examples include pET32, pET41, pET43. The additional expressible polynucleotide may also encode one or more chaperones to facilitate correct protein folding. Furthermore, the translational fusion described above may encode a fusion molecule of the invention as will be described elsewhere in this specification.

In another embodiment, the present invention relates to a non-human host transfected or transformed with the vector of the invention.

Non-human hosts according to the invention can be single cells or multi-cellular organisms.

In a more preferred embodiment, the non-human host is a cell.

Suitable prokaryotic host cells comprise e.g. bacteria of the species Escherichia, such as strains derived from BL21 (e.g. BL21(DE3), BL21(DE3)PlysS, BL21(DE3)RIL, BL21(DE3)PRARE) or Rosetta®, Streptomyces, Salmonella or Bacillus. Suitable eukaryotic host cells are e.g. yeasts such as Saccharomyces cerevisiae or Pichia pastoris or insect cells such as Drosophila S2 or Spodoptera Sf9 cells.
Mammalian host cells that could be used include human Hela, HEK293, H9 and Jurkat cells, mouse NIH3T3 and C127 cells, COS 1, COS 7 and CV1, quail QC1-3 cells, mouse L cells, Bowes melanoma cells and Chinese hamster ovary (CHO) cells. Also within the scope of the present invention are primary mammalian cells or cell lines. Primary cells are cells which are directly obtained from an organism. Suitable primary cells are, for example, mouse embryonic fibroblasts (MEF), mouse primary hepatocytes, cardiomyocytes and neuronal cells as well as mouse muscle stem cells (satellite cells) and stable, immortalized cell lines derived therefrom. The recombinant peptide of the invention can be expressed in stable cell lines that contain the gene construct integrated into a chromosome or, in stable or transiently transfected cells, in the form of a plasmid.

Transgenic non-human animals as hosts transfected with, e.g. a gene gun, and/or expressing the nucleic acid molecule of the present invention also lie within the scope of the invention. In a preferred embodiment, the transgenic animal is a mammal, e.g. a hamster, mouse, rat, cow, cat, pig, dog, horse, rabbit or monkey. Transgenic plants as hosts transfected with and/or expressing the nucleic acid molecule of the present invention also lie within the scope of the present invention.

In yet another embodiment, the present invention relates to a method of producing a peptide of the invention comprising culturing the host cell of the invention under suitable conditions and isolating the peptide produced.

A large number of suitable methods exist in the art to produce peptides in appropriate hosts. If the host is a unicellular organism such as a prokaryote or a mammalian or insect cell, the person skilled in the art can revert to a variety of culture conditions. Conveniently, the produced protein is harvested from the culture medium, lysates of the cultured cells or from isolated (biological) membranes by established techniques. In the case of a multicellular organism as a host comprising multiple cells carrying the nucleic acid of the invention, a fraction of these cells may serve as source for the peptide of the invention, for example said fraction may be the harvestable part of a plant. A preferred method involves the synthesis of nucleic acid sequences c by PCR and its insertion into an expression vector. Subsequently a suitable host may be transfected or transformed with the expression vector. Thereafter, in the case that the host is a cell, the host is cultured to produce the desired peptide, which is isolated and purified.

Appropriate culture media and conditions for the above-described host cells are known in the art. For example, suitable conditions for culturing bacteria are growing them under aeration in Luria Bertani (LB) medium. To increase the yield and the solubility of the expression product, the medium can be buffered or supplemented with suitable additives known to enhance or facilitate both. E. coli can be cultured from 4 to about 37 °C, the exact temperature or sequence of temperatures depends on the molecule to be overexpressed. In general, the skilled person is also aware that these conditions may have to be adapted to the needs of the host and the requirements of the peptide or protein expressed. In case an inducible promoter controls the nucleic acid of the invention in the vector present in the host cell, expression of the polypeptide can be induced by addition of an appropriate inducing agent. Suitable expression protocols and strategies are known to the skilled person.

Depending on the cell type and its specific requirements, mammalian cell culture can e.g. be carried out in RPMI or DMEM medium containing 10% (v/v) FCS, 2mM L-glutamine and 100 U/ml penicillin/streptomycin. The cells can be kept at 37 °C in a 5% CO₂, water saturated atmosphere.
Suitable media for insect cell culture is e.g. TNM + 10% FCS or SF900 medium. Insect cells are usually grown at 27 °C as adhesion or suspension culture.
Suitable expression protocols for eukaryotic cells are well known to the skilled person and can be retrieved e.g. from in Sambrook, 2001.
Such methods are well known in the art (see, e.g., Sambrook et al., supra).

An alternative method for producing the peptide of the invention is in vitro translation of mRNA. Suitable cell-free expression systems for use in accordance with the present invention include rabbit reticulocyte lysate, wheat germ extract, canine pancreatic microsomal membranes, *E. coli* S30 extract, and coupled transcription/translation systems such as the TNT-system (Promega). These systems allow the expression of recombinant peptides or proteins upon the addition of cloning vectors, DNA fragments, or RNA sequences containing coding regions and appropriate promoter elements.

Methods of isolation of the peptide produced are well-known in the art and comprise, without limitation, method steps such as ion exchange chromatography, gel filtration chromatography (size exclusion chromatography), affinity chromatography, high pressure liquid chromatography (HPLC), reversed phase HPLC, disc gel electrophoresis or immunoprecipitation (see, for example, Sambrook, 2001).

The present invention also relates to a peptide comprising the amino acid sequence encoded by a nucleic acid molecule of the present invention or obtainable by the method of the present invention.

The peptide of the present invention can also be produced synthetically. Chemical synthesis of peptides is well known in the art. Solid phase synthesis is commonly used and various commercial synthesizers are available, for example automated synthesizers by Applied Biosystems Inc., Foster City, CA; Beckman; MultiSyntech, Bochum, Germany etc. Solution phase synthetic methods may also be used, although it is less convenient. By using these standard techniques, naturally occurring amino acids may be substituted with unnatural amino acids, particularly D-stereoisomers, and also with amino acids with side chains having different lengths or functionalities. Functional groups for conjugating to small molecules, label moieties, peptides, or proteins or for purposes of forming cyclized peptides may be introduced into the molecule during chemical synthesis. In addition, small molecules and label moieties may be attached during the synthetic process. Preferably, introduction of the functional groups and conjugation to other molecules minimally affects the structure and function of the subject peptide.
The N-and C-terminus may be derivatized using conventional chemical synthetic methods. The peptides of the invention may contain an acyl group, such as an acetyl group.
Methods for acylating, and specifically for acetylating the free amino group at the N-terminus are well known in the art. For the C-terminus, the carboxyl group may be modified by esterification with alcohols or amidated to form-CONH₂ or CONHR. Methods of esterification and amidation are done using well known techniques.

Furthermore, the peptide of the invention may also be produced semi-synthetically, for example by a combination of recombinant and synthetic production. In the case that fragments of the peptide are produced synthetically, the remaining part of the peptide would have to be produced otherwise, e.g. recombinantly, and then linked to the fragment to form the peptide of the invention.

The present invention furthermore relates to a fusion molecule comprising the peptide of the invention.

A fusion molecule, in the context of the present invention is an at least bipartite molecule comprising the peptide of the invention forming one moiety coupled to at least one other moiety, as has been defined above.

The fusion molecule of the present invention can be produced and isolated according to the methods described above for the production of the peptide of the invention.

In a preferred embodiment, the peptide is fused to a nucleic acid, a peptide or a polypeptide, an aptamer, a small molecule, a nanoparticle or nanocarrier or a contrast agent.
The nucleic acid can be linear or circular, e.g. in the form of a plasmid, an antisense RNA or siRNA. In this regard, the above described mucleic acid mimicking molecules are also suitable as moieties. Aptamers are DNA or RNA molecules that have been selected from random pools based on their ability to bind other molecules. Aptamers include those which bind nucleic acid, proteins, small organic compounds, and even entire organisms such as unicellular organisms. A database of aptamers is maintained at http://aptamer.icmb.utexas.edu/.
More specifically, aptamers can be classified as DNA or RNA aptamers or peptide aptamers. Whereas the former consist of (usually short) strands of oligonucleotides, the latter consist of a short variable peptide domain, attached at both ends to a protein scaffold.

Nanoparticles (or nanopowders or nanoclusters or nanocrystals or nanocarriers) are microscopic particles with at least one dimension less than 100 nm. Nanocarriers, usually having a diameter of about 50 to 500 nm, are able to house smaller molecules, especially drugs which can then be delivered at the desired site. Nanocarriers existing to date are temperature and/or pH sensitive, which means that they can release their cargo upon heating or a change in the pH. They are mostly structurally stable in the normal physiological environment and resistant to e.g. intravenous administration. Polymeric core-shell nanocarriers are small in size (generally less than 200 nm), with shells that protect enclosed bioactive compounds against degradation and digestive fluids.

In a preferred embodiment of the fusion molecule of the present invention, the peptide of the invention is fused to a reporter unit. Suitable reporter units are well-known to the skilled person and comprise fluorescent dyes such as FITC or TAMRA or reporter units for MRI or PET such as Gd-DOTA-, Gd-DTPA-, ⁶⁴Cu-DOTA- or ⁶⁸Ga-DOTA, or nucleic acids such as siRNA, antisense oligonucleotides or nucleic acids encoding reporter genes, preferably operably linked to a regulatory element, such as a promoter, or contrast agents for other imaging techniques.

In another preferred embodiment of the fusion molecule of the present invention, the peptide of the invention is fused to a pharmacologically active compound. Pharmacologically active compounds may belong to different substance classes such as e.g. nucleic acids, peptides, small molecules, nanoparticles etc. as listed above.

In case the peptide of the invention is fused to more than one other moiety, suitable combinations of moieties can be e.g. a linker moiety and a pharmacologically active moiety, or a detectable moiety and a pharmacologically active moiety, optionally further comprising one or more linker moieties.

In a preferred embodiment, the peptide or fusion molecule is applied for research purposes.
The peptide or fusion molecule of the present invention can be applied e.g. in optical imaging methods, magnetic resonance imaging (MRI), primarily used in medical imaging to demonstrate pathological or other physiological alterations of living tissues, or positron emission tomography (PET), a nuclear medicine medical imaging technique which produces a three-dimensional image or map of functional processes in the body.
In MRI, a contrast agent may be applied to improve the measurement. The agent may be as simple as water, taken orally, e.g. for imaging the stomach and small bowel although substances with specific magnetic properties may also be used. Most commonly, a paramagnetic contrast agent (usually a gadolinium compound) is given. This provides high sensitivity for e.g. the detection of vascular tissues (e.g. tumors) and permits assessment of brain perfusion (e.g. in stroke).
More recently, superparamagnetic contrast agents (e.g. iron oxide nanoparticles) have become available. These may be used for liver imaging - normal liver tissue retains the agent, but abnormal areas (e.g. scars, tumors) do not. They can also be taken orally, to improve visualisation of the gastrointestinal tract, and to prevent water in the gastrointestinal tract from obscuring other organs (e.g. pancreas).
Diamagnetic agents such as barium sulfate have been studied for potential use in the gastrointestinal tract, but are less frequently used.
To conduct a PET scan, a short-lived radioactive tracer isotope, which decays by emitting a positron and which also has been chemically incorporated into a metabolically active molecule, is injected into the living subject (usually into the blood circulation). During a waiting period, the metabolically active molecule becomes concentrated in the tissues of interest; then the research subject or patient is placed in the imaging scanner. The molecule most commonly used for this purpose is fluorodeoxyglucose (FDG), a sugar, for which the waiting period is typically an hour.
The peptide of the present invention may serve as shuttle in order to target the respective contrast agent coupled thereto to the site of interest in both methods. The intracellular delivery should provide a higher sensitivity as well as specificity as compared to known commercially available contrast agents. Aside of the more specific detection of targeted cells (e.g. in cancer diagnosis or the detection of loss of β-cells in diabetes) such contrast agents can be used for non-invasive tracking of the fate and action of transplanted cells in cellular therapies (e.g. cancer treatment with cytotoxic T-cells). Cell based therapies such as stem cell therapies or adoptive immunotherapies were already successfully tested and also monitored in animal models, mainly by invasive optical imaging methods (e.g. Sauer MG, 2004). However, the clinical application still suffers from the lack of a non-invasive diagnostic method for a long-term quantitative and qualitative evaluation of the transplanted cells. This is essential for the monitoring of the treatment and its efficacy. By a non-toxic labeling procedure (most preferable repeatedly applicable) with specifically targeted contrast agents cells can be followed in vivo, and their accumulation as well as function can be monitored using imaging techniques like MRI, nuclear or optical imaging.

The present invention furthermore relates to a pharmaceutical composition comprising the peptide of the invention or the fusion molecule of invention.

The term "composition", as used in accordance with the present invention, relates to a composition which comprises at least one of the recited compounds. The composition may be in solid or liquid form and may be, inter alia, in the form of (a) powder(s), (a) tablet(s), (a) solution(s) or (an) aerosol(s).

In accordance with the present invention, the term "pharmaceutical composition" relates to a composition for administration to a patient, preferably a human patient. The pharmaceutical composition of the invention comprises the compounds recited above, alone or in combination. It may, optionally, comprise further molecules capable of altering the characteristics of the compounds of the invention thereby, for example, stabilizing, modulating and/or activating their function. The pharmaceutical composition of the present invention may, optionally and additionally, comprise a pharmaceutically acceptable carrier. By "pharmaceutically acceptable carrier" is meant a non-toxic solid, semisolid or liquid filler, diluent, encapsulating material or formulation auxiliary of any type. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by well known methods. Generally, the formulations are prepared by contacting the components of the pharmaceutical composition uniformly and intimately with liquid carriers or finely divided solid carriers or both. Then, if necessary, the product is shaped into the desired formulation.

In a preferred embodiment, the composition is suitable for parenteral administration. The term "parenteral" as used herein refers to modes of administration, which include intravenous, intramuscular, intraperitoneal, intrasternal, subcutaneous and intraarticular injection and infusion. These pharmaceutical compositions will be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.01 mg to 50 mg and most preferably 0.01 mg to 10 mg per day.

The components of the pharmaceutical composition to be used for therapeutic administration must be sterile. Sterility is readily accomplished by filtration through sterile filtration membranes (e.g., 0.2 micron membranes).

The components of the pharmaceutical composition ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic Water-for-Injection.
Preservatives and other additives may also be present such as, for example, antimicrobials, anti oxidants, chelating agents, and inert gases and the like. Furthermore, the pharmaceutical composition may comprise further agents depending on its intended use.

The pharmaceutical composition may be particularly useful for the treatment and/or prevention of diseases, including for example cancer, enzyme deficiency diseases, infarcts or cerebral ischemia, diabetes, inflammatory diseases, infections such as bacterial, viral or fungal infections, autoimmune diseases such as systemic lupus erythematodes (SLE), multiple sclerosis or rheumatoid arthritis, diseases with amyloid-like fibrils such as Alzheimer's disease (AD) and Parkinson's disease (PD) or certain forms of myopathy. Further preferred diseases are those where the pharmaceutical composition has to cross restrictive membrane structures such as the blood-brain barrier in order to be effective. Examples of such diseases are AD or PD.

Cancer, in accordance with the present invention refers to a class of diseases or disorders characterized by uncontrolled division of cells and the ability of these to spread, either by direct growth into adjacent tissue through invasion, or by implantation into distant sites by metastasis (where cancer cells are transported through the bloodstream or lymphatic system).

Enzyme deficiencies leading to disorders are caused by one or more mutations in one or more enzymes in a cell including fatty acid oxidation disorders, urea cycle disorders, phenylketonuria, glycogen branching enzyme deficiency and mitochondrial enzyme deficiencies.

Bacterial infections, in accordance with the present invention include but are not limited to bacterial meningitis, cholera, diphtheria, listeriosis, pertussis (whooping cough), pneumococcal pneumonia, salmonellosis, tetanus, typhus or urinary tract infections.

Viral infections, in accordance with the present invention include but are not limited to mononucleosis, AIDS, chickenpox, common cold, cytomegalovirus infection, dengue fever, Ebola hemorrhagic fever, hand-foot and mouth disease, hepatitis, influenza, mumps, poliomyelitis, rabies, smallpox, viral encephalitis, viral gastroenteritis, viral encephalitis, viral meningitis, viral pneumonia or yellow fever.

Fungal infections in accordance with the present invention include but are not limited to aspergillosis, blastomycosis, candidiasis, coccidioidomycosis, cryptococcosis, histoplasmosis or tinea pedis.

Autoimmune diseases, in accordance with the present invention refer to diseases which arise from an overactive immune response of the body against substances and tissues normally present in the body. Autoimmune diseases are well known to the person skilled in the art and include, but are not limited to Lupus erythematosus, acute disseminated encephalomyelitis, aplastic anemia, autoimmune hepatitis, diabetes mellitus, multiple sclerosis, optic neuritis or rheumatoid arthritis.

Diseases with amyloid-like fibrils in accordance with the present invention are diseases which share as a common feature that the normally soluble peptide amyloid-beta or the protein alpha-synuclein aggregates into an ordered fibrillar structure typically resulting in increased oxidative injury, excitotoxicity and altered cell cycle. Diseases with amyloid-like fibrils include but are not limited to Alzheimer's disease (AD) and Parkinson's disease (PD).

Alzheimer's disease is a neurodegenerative disease characterized by progressive cognitive deterioration together with declining activities of daily living and neuropsychiatric symptoms or behavioral changes. It is the most common type of dementia.

Parkinson's disease is a degenerative disorder of the central nervous system that often impairs the sufferer's motor skills and speech.

Myopathies are neuromuscular diseases in which the muscle fibers do not function, resulting in muscular weakness. Several classes of myopathy are known and include but are not limited to for example muscular dystrophies, congenital myopathies, mitochondrial myopathies or inflammatory myopathies.

In a preferred embodiment of the present invention, the pharmaceutical composition is used as a vaccine to prevent, inter alia, bacterial, viral or fungal infections as described above. In a particularly preferred embodiment, the vaccine is a DNA vaccine. Accordingly, the peptide of the invention is coupled to a nucleic acid which, when introduced into a cell, will be translated and the resulting peptide or protein will be processed in order to be presented in the form of antigenic fragments on the surface of cells, wherein the latter are usually found in bacteria, fungi or viruses.
Alternatively, the composition of the present invention comprising the peptide of the invention coupled to another moiety, such as a peptide or protein, also directly provides the cell with the expressed protein/peptide or the molecule against which immunity is to be induced when introduced into a cell without the need for it to be translated.

In the treatment of the above disorders and diseases, at least one pharmacologically active moiety is to be coupled to the peptide of the invention. As described above for moieties in general, pharmacologically active moieties may belong to different substance classes.

In addition to a pharmacologically active moiety, the fusion molecule of the invention may further comprise a targeting sequence or moiety specifically targeting to the desired tissue or structure as described further below.

In addition, the present invention relates to a diagnostic composition comprising at least one of (a) the nucleic acid molecule of the invention, (b) the vector of the invention, (c) the peptide of the invention, or (d) the fusion molecule of the invention.

In accordance with the present invention, the term "diagnostic composition" relates to compositions for diagnosing individual patients for their potential response to or curability by the pharmaceutical compositions of the invention. Furthermore, a diagnostic composition can denote a substance comprising the peptide of the invention fused to at least one more moiety for research purposes as has already been described above for the peptide and the fusion molecule of the invention. The diagnostic composition may further comprise appropriate buffer(s). The diagnostic composition may be packaged in a container or a plurality of containers.

Preferred embodiments of the diagnostic composition comprise the peptide of the present invention fused to a reporter unit and a target sequence specifically targeting to the desired tissue or structure. For example, the target sequence can be an antisense oligonucleotide or a peptide sequence which is enzymatically cleavable. Such target sequences can be designed in order to deliver the reporter units to e.g. cancerous cells or tissues or otherwise diseased cells or tissues.
Using the diagnostic composition of the present invention, diseases such as cancer can be detected in an early stage thus enabling for an early and more promising therapy. In particular, the pH value in cancerous tissue is usually more acidic than in non-cancerous tissue. Accordingly, a reporter unit sensitive to changes in the pH value and thus capable of detecting tissue with an e.g. more acidic pH value, in connection with the diagnostic agent of the present invention may serve in the detection of cancer.
Another example refers to the detection of insulin mRNA in insulin producing β-cells by coupling the peptide of the present invention to an antisense nucleic acid binding to insulin mRNA. With this molecule as a diagnostic tool, the insulin production in β-cells can be monitored and the decrease of insulin production and/or loss of β-cells which may indicate an early stage of diabetes can be detected.
Yet a different example refers to the detection of transplanted, healthy cells in the body after a transplantation event to monitor the tolerance of the body towards the graft as well as the fate of transplanted cells in the body.

The above-described combinations of moities usable in the detection and/or diagnosis with therapeutically applicable moieties become more and more important in the context of theranostics. This is the term used to describe the proposed process of diagnostic therapy for individual patients, to test them for possible reaction to taking a new medication and to tailor a treatment for them based on the test results.

The present invention furthermore relates to a method of detecting the internalization behaviour of a fusion molecule comprising the peptide of the invention or a fusion molecule according to the invention, comprising (a) administering said fusion molecule to a cell and (b) detecting the internalization of the fusion molecule.

The present method is particularly suitable to estimate the applicability of the peptide of the present invention in connection with other moieties coupled thereto for medical or research purposes, e.g. in the form of pharmaceutical or diagnostic compositions. If efficient internalization of the peptide or the fusion molecule comprising more than one moieties is detected and, optionally, its localization is in the cytoplasm, this indicates that the respective molecule can be efficiently used for medical and research purposes.

In addition, the present invention relates to a method of treating, preventing or diagnosing a condition selected from cancer, enzyme deficiency diseases, infarcts, cerebral ischemia, diabetes, inflammatory diseases, infections such as bacterial, viral or fungal infections, autoimmune diseases such as systemic lupus erythematodes (SLE) or rheumatoid arthritis, diseases with amyloid-like fibrils such as Alzheimer's disease (AD) and Parkinson's disease (PD) or certain forms of myopathy comprising administering the composition of the invention to a subject in need thereof.

The present invention also relates to the peptide, the fusion molecule or the composition of the invention for therapeutic or diagnostic purposes. In a preferred embodiment the therapeutic purpose is the treatment and/or prevention of cancer, enzyme deficiency diseases, infarcts, cerebral ischemia, diabetes, inflammatory diseases, infections such as bacterial, viral or fungal infections, autoimmune diseases such as systemic lupus erythematodes (SLE) or rheumatoid arthritis, diseases with amyloid-like fibrils such as Alzheimer's disease (AD) and Parkinson's disease (PD) or certain forms of myopathy.

The present invention furthermore relates to a kit comprising at least one of (a) the nucleic acid molecule of the invention, (b) the vector of comprising the nucleic acid molecule of the invention, (c) the host cell comprising the vector of the invention, (d) the (poly)peptide of the invention or (e) the fusion protein of the invention.

The various components of the kit may be packaged in one or more containers such as one or more vials. The vials may, in addition to the components, comprise preservatives or buffers for storage.

The figures show:
**Figure 1****:** Comparison of the intracellular fluorescence of the synthesized peptide fragments (cargo: K(FITC), peptide 23 = SEQ ID NO: 2). corr. f.u. = corrected fluorescence units (measured cell-related FITC fluorescence corrected for the number of cells evaluated by Bisbenzimid 33342 fluorescence.

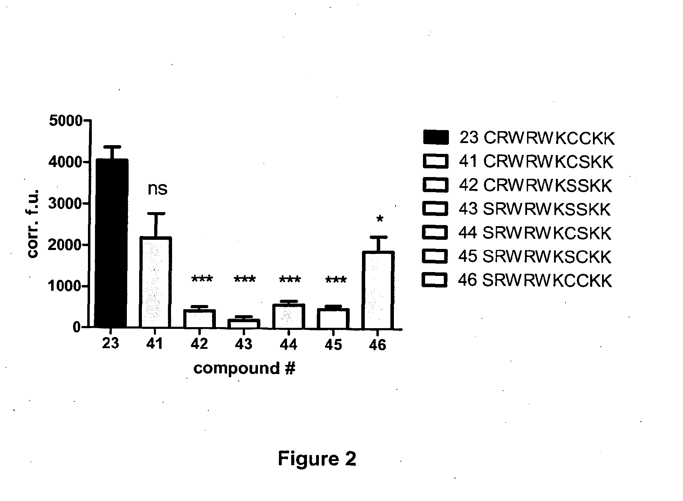

**Figure 2****:** Influence of substitution of cysteines by serine in peptide 23 (SEQ ID NO: 2) on the internalization efficiency. ns, not significant; *, p<0.05, ***, p<0.001 significantly different compared to peptide 23 (ANOVA, Bonferroni's Multiple Comparison Test). K(FITC) was coupled to all peptides at the N-terminus.

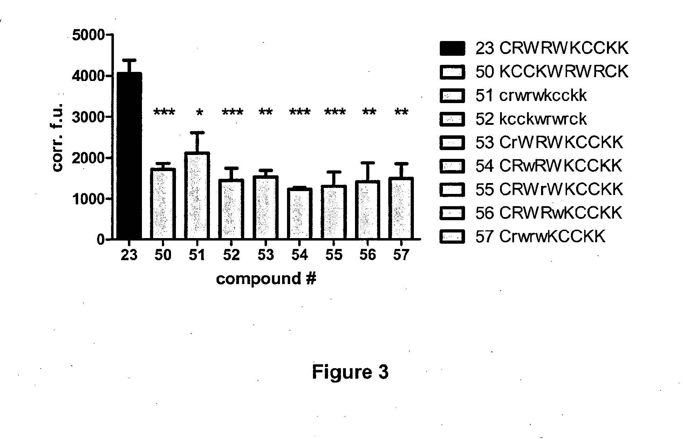

**Figure 3****:** Influence of sequence order and amino acid chirality on the internalization efficiency. *, p<0.05, **, p<0.01, ***, p<0.001 significantly different compared to peptide 23 (ANOVA, Bonferroni's Multiple Comparison Test). K (FITC) or k (FITC) was coupled to all peptides at the N-terminus.

The invention will now be described by reference to the following examples which are merely illustrative and are not to be construed as a limitation of scope of the present invention.

### Example 1: Peptide Synthesis: materials and method

All solvents were of peptide synthesis grade. N,N-dimethylformamide (DMF), dichloromethane (DCM), trifluoroacetic acid (TFA), fluorescein isothiocyanate (FITC) and methanol were purchased from Acros Organics, Belgium. Protected Fmoc amino acid as well as resin was obtained from Novabiochem (Nottingham, UK). The side chain of lysine was protected by Boc or Dde, Cys by Trt or tBu, Arg by Pbf and, Trp by Boc.

### (A) Automatic Peptide Synthesis:

Peptides were prepared by fully automated solid-phase peptide synthesis using Fmoc/tBu-strategy and α-Fmoc-(ε-BOC)-lysine-TCP-polystyrene resin. The resin was distributed in 30 mg aliquots (15 µmol) to filter tubes, which were positioned in the format of a microtiter plate on valve blocks. Fmoc deprotection were carried out two times, 10 min. each, with 30 % piperidine in dimethylformamide (DMF) (300 µl). Nine washing steps were done with DMF (300 µl). Fmoc-amino acids (0.5 M) were dissolved with 1-hydroxybenzotriazole (HOBt) (0.5 M) in DMF. Diisopropylethylamine [3 M in NMP, 60 µl] was added to the reaction vessels. Coupling reagents diisopropylcarbodiimide [3 M in DMF , 50 µl, first coupling] or 1-H-benzotriazolium-1-[bis(dimethylamino)methylene]-5-chloro-tetrafluoroborate (1-), 3-oxide [TCTU, 0.5 M in DMF, 200 µl, second coupling] and Fmoc-amino acids [first coupling 200 µl; second coupling 100 µl] were distributed to the reaction vessels. After 1 h coupling time, coupling reagents were filtered off and the resins were washed with DMF [1 x 200 µl] followed by a second coupling step (1 h). Amino acids were introduced using a sevenfold molar excess of the respective Fmoc-L-amino acid. After washing steps [4 x 400 µl] Fmoc deprotection was carried out two times, 10 min. each, with 30 % piperidine in DMF (300 µl). Nine washing steps were done with DMF (300 µl). The coupling process was repeated according to the length of the peptide. The ε-group of N-terminal lysine was labeled manually with FITC (4 fold excess) with triethylamine (1:2) in DMF overnight. Peptides were washed with DMF, DCM, methanol (4x each) and dried before cleaving from the resin. The peptides were cleaved off the resin and side-chain deprotected with Reagent K (500 µl).

### (B) Manual Peptide Synthesis:

The synthesis of selected peptides was carried out by solid phase Fmoc chemistry using a manual multiple peptide synthesizer (Heidolph Synthesis 1 synthesizer). Peptides were synthesized on α-Fmoc-(ε-BOC)-lysine-2-chlorotrityl resin (0.83 mmol/g). Fmoc protected amino acids (4 fold excess) were coupled with DIC/ HOBT activation for 60 min. Fmoc was removed by 30% piperidine in DMF (2 x 10 min). Resin was washed after each cycle of coupling and deprotection with DMF (4x). Completeness of coupling and deprotection was monitored by Kaiser Test (38). Peptides were washed with DMF, DCM, methanol (4x each) and dried. The peptides were cleaved off the resin and side-chain deprotected with Reagent K (500 µl).
This cleavage reaction was optimized further on, in particular for the synthesis of DOTA based compounds, by using TFA: Reagent K: TMSBr (8.5:1.45:0.05) for 2 hr instead of Reagent K alone. For the oxidized peptides a different cleavage cocktail consisting of TFA:TIPS:H₂O was used for 1 h followed by addition of DMSO (5:1) at 0°C for 30 min and stirring for 1 h at RT.

### Example 2: Purification and characterization:

All peptides were precipitated with MTBE. Precipitates were collected by centrifugation and resuspended in cold MTBE 2 times and were lyophilized. Samples were purified by semipreparative RP-HPLC at RT using water/0.1% TFA (solvent A) and Acetonitrile/0.1% TFA (solvent B) on a Varian Polaris C18-Ether column (21.2 mm in diameter, length of 250 mm, particle size 5 µm). The product containing fractions were defined by investigation with analytical RP-HPLC on a Varian Polaris C18-Ether column (4.6mm in diameter, length of 250 mm, particle size 5 µm). Mass spectrometry (ESI-MS) was used for further characterization. The purified product was dissolved in water and tert.butyl alcohol (1:4) with 2% acetic acid, and lyophilised.
Peptides containing cysteine are prone to oxidative formation of disulphide bonds, which could be formed intramolecularly, resulting in a cyclic peptide, or intermolecularly, forming oligomers or aggregates.
As this CPP is a cysteine rich peptide, care has to be taken during synthesis and storage of the peptides. During the synthesis of peptide, choice of protecting groups and proper scavengers during cleavage should be optimized. After lyophilization peptides were stored under nitrogen. Resolved samples for internalization studies were aliquoted and stored at -80°C. Solutions for cell studies were freshly prepared for each experiment from these aliquots.

### Example 3: SAR studies

Various fragments derived from the proposed fragment (Cro₂₇₋₃₉) were synthesized to achieve an optimal fragment maintaining the distinctive features of the parent sequence. The original fragment Cro₂₇₋₃₉ (peptide 1, see table 1) is a peptide of 13 amino acid including synthetically difficult amino acids like methionine or cysteine. The aim of this study was to simplify the sequence maintaining the distinctive features of the parent sequence Cro₂₇₋₃₉. To visualize this aim series of peptides were synthesized by substituting or deleting amino acid residues but preserving the basic amino acids in the sequence. K (FITC) or k (FITC) was coupled to all peptides at the N-terminus to measure the internalization efficiency.

### (A) Substitution of cysteine:

The purpose of substitution was to understand the importance of cysteine in the sequence. Replacement of cysteines one by one or all together by aminobutyric acid or serine will help in estimating the number and position of cysteines responsible for maintaining penetration ability and to minimize the complexity of the synthesis by avoiding cysteines.

### (B) Deletion of cysteines:

The same rationale was valid studying the possibilities of avoiding cysteines completely. In the way of doing so we tried to delete cysteines from the sequence one by one and studying the effect of the deletion on the internalization efficiency.

### (C) Shortening the sequence:

To find the minimal amino acid composition necessary for the best uptake, combinations were designed by deleting amino acids from the N-terminus keeping the positively charged amino acids the same. On the other hand, deletion of the positively charged amino acids from C-terminus was also studied.

### (D) Shortening the sequence and substitution:

Other variations include combined substitution and deletion to study the combined effect of the two. This included substitution of the cysteines by amino butyric acid and deletion from N-terminal amino acids one by one (until reaching the first cysteine). The idea was to check the appropriate length and the number of cysteine residues needed.

### (E) Substitution of tryptophan:

Tryptophans are known to be prone to side reactions like alkylation. Also blends of tryptophans and cysteines are not the most favorable ones as there are more chances of side reactions during the course of disulphide formation by various methods chemically. Therefore tryptophan was substituted by proline or phenylalanine one by one or completely.

### (F) Effect of chirality:

Screening of a number of fragments for cellular uptake led to a peptide shorter in length (peptide 23, SEQ ID NO: 2). Studies were carried on this peptide in order to check factors other than the amino acid composition itself.
Other distinctive features are the influence of the chirality of the peptide backbone as well as the sequence order on the cellular uptake. Peptides with d-isomers (whole sequence with D-amino acids), L-amino acids in reversed order and with D-amino acids in reversed order were synthesized. In addition, the effect of stereochemistry was studied only in the region of Trp-Arg-Trp-Arg by using single D-amino acids or exchanging all four residues. This pattern seemed to be important as indicated by the results of the initial screening.

### (G) Introduction of a spacer unit:

Studies show the importance of cysteines in the proposed fragment and their oxidation status. To extend our study in this direction attempts were made focusing on oxidation of the proposed fragment. In SEQ ID NO: 2 two cysteines are adjacent to each other. Investigations were carried out to estimate the favorable conditions for disulphide bond formation between them. The purpose of introduction of the spacer glycine was not only to study the necessity of an optimal distance between two cysteines for disulphide linkage but also to facilitate the synthesis of defined disulphide bonds through fragment condensation. Additionally, the effect of elongating the sequence by introducing an additional cysteine was studied for the convenient synthesis of peptides with two defined disulphide linkages.

Results of the experiments are depicted in Figures 1 to 3.

### Example 4: Effect of cargos on uptake

CPPs are known to be the carrier of various cargos through the plasma membrane. Cellular uptake of various cargos was studied. Cargos were linked to the peptide through a linker which can be varied according to the need. In this study lysine is used as a linker. Choice of the amino protecting groups depends on the requirement of the synthesis.

### Fluorescent dye as a cargo:

➢ *Coupling of FITC to* ε*-amino group:*
   For structure activity studies all peptides were coupled to FITC through a Boc-Lysine (Fmoc)-OH as a linker. The ε-group of N-terminal lysine was labeled with FITC (4 fold excess) with triethylamine (1:2) in DMF overnight.
   Analysis and purification conditions are same as before (C).
➢ *Coupling of TAMRA and carboxyfluorescein:*
   Different dyes were coupled at ε-amino group to study the effect of cargo on internalization. Different colored dyes were synthesized for the chasing experiments to study the mechanism of internalization. TAMRA was coupled both at α-amino and ε-amino group to study the effect of the position of the cargo on internalization. Coupling of these dyes were done by DIC, HOBt activation for 8h.
   Analysis and purification conditions are same as before (C).

### MR reporter as cargo:

➢ *Gd(DOTA) as cargo:*
   To extend the variations in the range of the cargos an attempt was made for the synthesis of bimodal intracellular contrast agents using proposed peptide as a carrier of Gd (DOTA) [as MR reporter ] and FITC [fluorescent marker].
   Synthesis of the peptide was done as mentioned before. Coupling of the cargo was carried out by coupling of Fmoc-Lys (Dde)-OH as the linker. DOTA was coupled by DIC, HOBt activation for 12 h at α-amino position (after Fmoc deprotection). Later FITC was coupled at ε-amino after Dde deprotection by 2% hydrazine in DMF 3min X 2. Analysis and purification conditions are same as before (C).
   The actual concentration of the peptide was estimated by fluorescence spectroscopy to be ∼ 25%. Accordingly GdCl₃.6H₂O was added (1:1) at pH 6 50°C for 18h. Repurification was done to get rid of the excess gadolinium.
   Analysis and purification conditions are same as before (C). Samples were dialyzed for 48h to remove excess Gadolinium and other inorganic salts.

### Example 5: Cell Culture

NIH 3T3 mouse fibroblast cells were cultured as a monolayer at 37°C with 10% CO₂ in Dulbecco's Modified Eagle's Medium (DMEM) supplemented with 10% fetal bovine serum, 4 mM L-glutamine, 100 µg/mL streptomycin and 100 U/mL penicillin (all purchased from Biochrom AG, Germany). Cells were passaged by trypsinization with trypsin/EDTA 0.05/0.02% (w/v) in phosphate-buffered saline (PBS; Biochrom AG, Germany) every second to third day.

### Example 6: Determination of concentrations of fluorescently labeled peptides

Peptides were dissolved in MilliQ water to obtain a 10mM solution by weight. For the determination of the exact peptide concentration, these stock solutions were diluted 1:100 in DMEM. The absorbance of the solutions was measured by multiplate reader (BMG Labtech, Germany) at 485nm with ratiometric correction of turbidity at 690nm. The concentrations of the stock solutions were calculated assuming ε_{carboxyfluorescein 485 nm}=81,000 l/(mol·cm). The concentrations of TAMRA labeled peptides was calculated assuming ε_{TAMRA 540 nm} =40,000 l/ (mol·cm).

### Example 7: Uptake Assay

Internalization experiments on cells were performed in 96 well microplates by inoculation of NIH 3T3 fibroblasts (1 × 10⁴ cells/well). After 24 h, cells were incubated with 2.5 µM of different peptide solutions in DMEM for additional 18 h at 37 °C with 10% CO₂. Before washing, cells were incubated with Bisbenzimid 33342 (Hoechst 33342), a nuclear stain, in order to estimate the cell number. Cells were washed with Hanks' buffered saline (Biochrom AG, Germany) and extracellular fluorescence was quenched by incubating with cold trypan blue 0.05% (w/v) in phosphate-buffered saline (Biochrom AG, Germany) for 3 min followed by repeated washes with Hanks' buffered saline. Cell-related FITC fluorescence (Ex 485 nm/Em 530 nm) and cell number (Ex 346nm/ Em 460nm) was evaluated in a multiplate reader. Experiments were run at least three times for each peptide with six replicates. Statistical analysis was performed by Student's *t*-test or ANOVA with Dunnett's post test. P values < 0.05 were considered significant. The results are depicted in Figures 1 to 3.

### Example 8: Microscopy

The same cells were subjected to microscopic studies without fixation using a Zeiss Axiovert 200 M (Germany) microscope with a LD Plan NeoFluor 40X objective. The imaging conditions were kept constant for the observation of different samples. Cellular localization and distribution of the peptide was observed by irradiating with blue light (470/40 nm) and observing at 525/50 nm. The bright punctate and encapsulated appearing FITC fluorescence was categorized as vesicular uptake while diffused fluorescence appeared to be distributed in the entire cell with similar intensity. Manual observation of at least three experiments was compiled to conclude if the peptide shows diffused, vesicular or both types of uptake. Apart from FITC fluorescence, the nuclear labeling by Hoechst was observed at 460/50 nm and trypan blue fluorescence viewed at 645/75 nm. Also phase contrast images with DIC of the same area were made to observe if the cells maintain their normal morphology in the presence of peptides.

### Example 9: Time Course

Cells were seeded at a density of 1 × 10⁴ cells/well in 96 well microplates. After 48 h, cells were incubated with 2.5 µM of peptide solution in DMEM for different time points (30 min - 18h) at 37°C. Labeling with Hoechst, quenching and washings were performed as explained in detail in uptake assay. Fluorescence spectroscopy and microscopy was performed on the plate.

### Example 10: Effect of temperature on uptake

Cells were seeded at a density of 1 × 10⁴ cells/well in 96 well microplates. After 48 h, cells were incubated with 2.5 µM of different peptide solutions in DMEM for additional 4 h at 37°C or 4°C. Labeling with Hoechst, quenching and washings were performed as explained in detail in uptake assay. Fluorescence spectroscopy and microscopy was performed on the plate.

### Example 11: Elucidating the mechanism of uptake

In order to understand the mechanism of internalization, cells were treated with a variety of inhibitors of uptake like wortmannin, methyl-β-cyclodextrin, NaN₃/2-deoxyglucose, chloroquine, etc. Cells were cultured at a density of 1 × 10⁴ cells/well in 96 well microplates. After 48 h, cells were pre-incubated with indicated inhibitors for 30 min followed by additional 4h co-incubation with 2.5 µM of different peptide solutions in serum free DMEM at 37 °C or 4°C. Labeling with Hoechst, quenching and washings were performed as mentioned in detail in uptake assay. Fluorescence spectroscopy and microscopy was performed on the plate.

### References

Altschul, S. F., Gish, W., Miller, W., Myers, E. W., Lipman, D.J., Basic local alignment search tool: J Mol Biol. 1990;215(3):403-10.
Altschul, S. F. and Gish, W. Methods Enzymol. 1996, 266:460
Ausubel, "Current Protocols in Molecular Biology", Green Publishing Associates and Wiley Interscience, N.Y. (1989)
Barnes, M., Freudenberg, J., Thompson, S., Aronow, B., Pavlidis, P., Experimental comparison and cross-validation of the Affymetrix and Illumina gene expression analysis platforms: Nucleic Acids Res. 2005 Oct 19;33(18):5914-23. Print 2005.
Bebbington, C.R., G. Renner, S. Thomson, D. King, D. Abrams, and G.T. Yarranton. 1992. High-level expression of a recombinant antibody from myeloma cells using a glutamine synthetase gene as an amplifiable selectable marker. Biotechnology (N Y). 10:169-75.
Beltran, J. R. et al, Saxs study of structure and conformational changes of crotamine: Biophys. J. 47 (1985) 33-35.
Boni-Mitake, M. et al ,Effects of 60Co gamma radiation on crotamine: Braz J Med Biol Res, December 2001, Volume 34(12) 1531-1538.
Boni-Mitake, M. et al , Distribution of 125I-labeled crotamine in mice tissues: Toxicon 48 (2006) 550-555.
Deshayes, S. et al, Cell-penetrating peptides: tools for intracellular delivery of therapeutics: Cell. Mol. Life Sci. 62 (2005) 1839-1849.
Casare, M. S. et al , Effects of 60Co radiation on the molecular strucure of crotamine: Radiation Physics and Chemistry 71 (2004) 417-418.
Derossi, D., Joliot, A.H., Chassaing, G., Prochiantz, A., The third helix of the Antennapedia homeodomain translocates through biological membranes: J Biol Chem. 1994;269(14):10444-50.
Egholm, M., Buchardt, O., Christensen, L., Behrens, C., Freier, S.M., Driver, D.A., Berg, R.H., Kim, S.K., Norden, B., Nielsen, P.E., PNA hybridizes to complementary oligonucleotides obeying the Watson-Crick hydrogen-bonding rules: Nature. 1993 Oct 7;365(6446):566-8.
Elmquist, A. et al, In vitro uptake and stability study of pVEC and its all-D analog: Biol Chem. 384 (2003) 387-93.
Fadel, V. et al, Automated NMR structure determination and disulfide bond identification of the myotoxin crotamine from Crotalus durissus terrificus: Toxicon 46 (2005) 759-767.
Fan, J.B., Hu, S.X., Craumer, W.C., Barker, D.L., BeadArray-based solutions for enabling the promise of pharmacogenomics. Biotechniques. 2005 Oct;39(4):583-8.
Fernandez-Carneado, J. et al, Potential Peptide Carriers: Amphipathic Proline-Rich Peptides Derived from the N-Terminal Domain of γ-Zein: Angew. Chem. Int. Ed. 43 (2004) 1811 -1814.
Fernandez-Carneado, J. et al, Fatty acyl moieties: improving Pro-rich peptide uptake inside HeLa cells: J. Peptide Res 65 (2005) 580-590.
Futaki, S. et al, Arginine-rich Peptides - An abundant source of membrane-permeable peptides having potential as carriers for intracellular protein delivery: J Biol Chem 276 (2001) 5836-5840.
Green, M., Loewenstein, P.M., Autonomous functional domains of chemically synthesized human immunodeficiency virus tat trans-activator protein. Cell. 1988 Dec 23;55(6):1179-88.
Gupta, B. et al, Intracellular delivery of large molecules and small particles by cell-penetrating proteins and peptides: Adv. Drug Delivery Rev. 57 (2005) 637- 651.
Hampe, O. G. et al, Model studies of crotamine self-association: Braz J Med Biol Res 22 (1989) 17-24.
Hampe, O. G. et al , Polyacrylamide gel electrophoretic studies on the self association of crotamine: Characterization and molecular dimension of n-mer species: Electrophoresis 11 (1990) 415-478.
Hampe, O. G. et al, An in vivo method for estimating the concentration of Crotamine in solution: J. Venom. Anim. Toxins 3 (1997) 33-36.
Henikoff, S., Henikoff, J.G., Amino acid substitution matrices from protein blocks.,Proc Natl Acad Sci U S A. 1992 Nov 15;89(22):10915-9.
Higgins and Hames (Eds.) "Nucleic acid hybridization, a practical approach" IRL Press Oxford, Washington DC, (1985)
Joliot, A., Pernelle, C., Deagostini-Bazin, H., Prochiantz, A., Antennapedia homeobox peptide regulates neural morphogenesis. Proc Natl Acad Sci U S A. 1991 Mar 1;88(5):1864-8.
Kerkis, A. et al , Properties of Cell Penetrating Peptides (CPPs) : IUBMB Life 58 (2006) 7-13.
Kerkis, A. et al, Crotamine is a novel cell-penetrating protein from the venom of rattlesnake Crotalus durissus terrificus: FASEB J 18 (2004) 1407-1409.
Lundberg, P. et al, A brief introduction to cell-penetrating peptides: J. Mol. Recognit. 16 (2003) 227-233.
Mae, M. and Langel, U., Cell-penetrating peptides as vectors for peptide, protein and oligonucleotide delivery: Curr Opin Pharmacol 6 (2006) 509-514.
Magzoub, M. et al, Cell-penetrating peptides: small from inception to application: Quart Rev Biophys 37 (2004) 147-195.
Mancin, A. C. et al , The analgesic activity of crotamine, a neurotoxin from Crotalus durissus terrificus (South American Rattle snake) venom : A biochemical and pharmacological study : Toxicon 36 (1998) 1927-1937.
Mascarenhas, Y. P. et al , Structure-function relationship for the highly toxic crotoxin from Crotalus durissus terrificus : Eur Biophys J 21 (1992) 199-205.
Matavel, A. C. S. et al, Tension generation and increase in voltage-activated Na+ current by crotamine: Eur J Pharmacol 348 (1998) 167-173.
Melikov, K. et al, Arginine-rich cell penetrating peptides: from endosomal uptake to nuclear delivery: Cell. Mol. Life Sci. 62 (2005) 2739-2749.
Michalsky, E. et al., Loops In Proteins (LIP) - a comprehensive loop database for homology modelling. Protein Eng. 16 (2003):979-985.
Mitchell, D.J. et al, Polyarginine enters cells more efficiently than other polycationic homopolymers: J Peptide Res 56 (2000) 318-325.
Murphy, G., M.I. Cockett, R.V. Ward, and A.J. Docherty. 1991. Matrix metalloproteinase degradation of elastin, type IV collagen and proteoglycan. A quantitative comparison of the activities of 95 kDa and 72 kDa gelatinases, stromelysins-1 and -2 and punctuated metalloproteinase (PUMP). Biochem J. 277 ( Pt 1):277-9.
Nascimento, F. D. et al ,Crotamine mediates gene delivery into cells through the binding to heparan sulfate proteoglycans: J. Biol. Chem (2007).
Nicastro, G. et al, Solution structure of crotamine, a Na+ channel affecting toxin from Crotalus durissus terrificus venom: Eur. J. Biochem. 270, 1969-1979 (2003).
Nielsen, P.E., Egholm, M., Berg, R.H., Buchardt, O., Sequence-selective recognition of DNA by strand displacement with a thymine-substituted polyamide. Science. 1991 Dec 6;254(5037):1497-500.
Novais, M. et al, 99mTc radiolabeling of crotoxin as a tool for biodistribution studies: J. Radioanalyt. Nucl. Chem.269 (2006) 591-595.
Pearson, W.R., Lipman, D.J., Improved tools for biological sequence comparison. Proc Natl Acad Sci U S A. 1988 Apr;85(8):2444-8.
Pooga, M., Soomets, U., Hällbrink, M., Valkna, A., Saar, K., Rezaei, K., Kahl, U., Hao, J.X., Xu, X.J., Wiesenfeld-Hallin, Z., Hökfelt, T., Bartfai, T., Langel, U., Cell penetrating PNA constructs regulate galanin receptor levels and modify pain transmission in vivo. Nat Biotechnol. 1998 Sep;16(9):857-61.
Oguiura, N. et al, New view on crotamine, a small basic polypeptide myotoxin from South American rattlesnake venom: Toxicon 46 (2005) 363-370.
Owens, G.C., Chappell, S.A., Mauro, V.P., Edelman, G.M., Identification of two short internal ribosome entry sites selected from libraries of random oligonucleotides. Proc Natl Acad Sci U S A. 2001 Feb 13;98(4):1471-6.
Rádis-Baptista, G. et al , Structure and chromosomal localization of the genefor crotamine, a toxin from the South American rattlesnake, Crotalus durissus terrificus: Toxicon 42 (2003) 747-752.
Rádis-Baptista, G., Oguiura, N., Hayashi, M.A., Camargo, M.E., Grego, K.F., Oliveira, E.B., Yamane, T., Nucleotide sequence of crotamine isoform precursors from a single South American rattlesnake (Crotalus durissus terrificus). Toxicon. 1999 Jul;37(7):973-84.
Richard, J. P. et al, Cell-penetrating Peptides: a Reevaluation of the mechanism of cellular uptake: J Biol Chem 278 (2003) 585-590.
Rothbard, J.B. et al, Arginine-Rich Molecular Transporters for Drug Delivery: Role of Backbone Spacing in Cellular Uptake: J. Med. Chem. 45 (2002) 3612-3618.
Sambrook et al., "Molecular Cloning, A Laboratory Manual"; CSH Press, Cold Spring Harbor, 1989
Sarin, V. K. et al, Quantitative monitoring of solid-phase peptide synthesis by the ninhydrin reaction: Anal.Biochem. 117 (1981) 147-157.
Sauer, M.G., Ericson, M.E., Weigel, B.J., Herron, M.J., Panoskaltsis-Mortari, A. Kren, B.T., Levine, B.L., Serody, J.S., June, C.H., Taylor, P.A., Blazar, B.R. A novel system for simultaneous in vivo tracking and biological assessment of leukemia cells and ex vivo generated leukemia-reactive cytotoxic T cells. Cancer Res. 2004,64:3914-3921.
Shen, R., Fan, J.B., Campbell, D., Chang, W., Chen, J., Doucet, D., Yeakley, J., Bibikova, M., Wickham Garcia, E., McBride, C., Steemers, F., Garcia, F., Kermani, B.G., Gunderson, K., Oliphant, A., High-throughput SNP genotyping on universal bead arrays. Mutat Res. 2005 Jun 3;573(1-2):70-82. Review.
Smith, T.F., Waterman, M.S., Identification of common molecular subsequences. J Mol Biol. 1981 Mar 25;147(1):195-7.
Soomets, U., Lindgren, M., Gallet, X., Hällbrink, M., Elmquist, A., Balaspiri, L., Zorko, M., Pooga, M., Brasseur, R., Langel, U., Deletion analogues of transportan. Biochim Biophys Acta. 2000 Jul 31;1467(1):165-76.
Steemers, F.J., Gunderson, K.L., Illumina, Inc. Pharmacogenomics. 2005 Oct;6(7):777-82.
Takeuchi, T. et al, Direct and Rapid Cytosolic Delivery Using Cell-Penetrating Peptides Mediated by Pyrenebutyrate: ACS Chem. Biol. 1 (2006) 299-303.
Thompson, J.D., Higgins, D.G., Gibson, T.J., CLUSTAL W: improving the sensitivity of progressive multiple sequence alignment through sequence weighting, position-specific gap penalties and weight matrix choice. Nucleic Acids Res. 1994 Nov 11;22(22):4673-80.
Toyama, M. H. et al, Biophysical, histopathological and pharmacological characterization of crotamine isoforms F22 and F32: Toxicon 41 (2003) 493-500.
Toyama, M. H. et al , Biochemical characterization of two crotamine isoforms isolated by a single step RP-HPLC from Crotalus durissus terrifcus (South American rattlesnake) venom and their action on insulin secretion by pancreatic islets: Biochim Biophy Acta 1474 (2000) 56-60.
Tréhin, R. and Merkle, H. P., Chances and pitfalls of cell penetrating peptides for cellular drug delivery: Eur J Pharm Biopharm 58 (2004) 209-223. Proc Natl Acad Sci U S A. 2000 Nov 21;97(24):13003-8
Wender PA, Mitchell DJ, Pattabiraman K, Pelkey ET, Steinman L, Rothbard JB., Zorko, M. et al, Cell-penetrating peptides: mechanism and kinetics of cargo delivery: Adv Drug Delivery Rev 57 (2005) 529- 545.

## Claims

1. A nucleic acid molecule encoding a peptide capable of being internalized into a cell, wherein said nucleic acid molecule comprises
(a) a nucleic acid molecule encoding a peptide having the amino acid sequence of SEQ ID NO: 2;
(b) a nucleic acid molecule having the DNA sequence of SEQ ID NO: 1, wherein T is U if the nucleic acid molecule is RNA;
(c) a nucleic acid molecule hybridizing under stringent conditions to the complementary strand of a nucleic acid molecule of (a) or (b), wherein the peptide encoded by said nucleic acid molecule has a cysteine at least at two positions selected from the group consisting of positions 1, 7 and 8 of SEQ ID NO: 2 and a positively charged amino acid at least at four positions selected from the groups consisting of position 2, 4, 6, 9 or 10 of SEQ ID NO: 2;
(d) a nucleic acid molecule encoding a peptide having at least 70% sequence identity with that of SEQ ID NO: 2, wherein at least at two positions selected from the group consisting of positions 1, 7 and 8 of SEQ ID NO: 2 a cysteine is present and wherein at least at four positions selected from the groups consisting of position 2, 4, 6, 9 or 10 of SEQ ID NO: 2 a positively charged amino acid is present; or
(e) a nucleic acid molecule degenerate with respect to the nucleic acid molecule of (c) or (d).

2. The nucleic acid molecule of claim 1 wherein the peptide further comprises a linker.

3. The nucleic acid molecule of.claim 2, wherein the linker is a lysine.

4. A vector comprising the nucleic acid molecule of any one of claims 1 to 3.

5. A non-human host transfected or transformed with the vector of claim 4.

6. The non-human host of claim 5 which is a cell.

7. A method of producing a peptide comprising culturing the host of claim 6 under suitable conditions and isolating the peptide produced.

8. A peptide encoded by the nucleic acid molecule of any one of claims 1 to 3 or produced by the method of claim 7.

9. A fusion molecule comprising the peptide of claim 8.

10. The fusion molecule of claim 9, wherein the peptide is fused to nucleic acid, a protein or peptide, an aptamer, a small molecule, a nanoparticle or nanocarrier or a contrast agent.

11. The fusion molecule of claim 9 or 10, wherein the peptide is fused to FITC, TAMRA, Gd-DOTA-, Gd-DTPA-, ⁶⁴Cu-DOTA-, ⁶⁸Ga-DOTA, siRNA, antisense oligonucleotides or nucleic acids encoding reporter genes.

12. A pharmaceutical composition comprising the peptide of claim 8 or the fusion molecule of any one of claims 9 to 11, optionally further comprising a pharmaceutically acceptable carrier, excipient and/or diluent.

13. The composition of claim 12 which is a vaccine.

14. A diagnostic composition comprising at least one of
(a) the nucleic acid molecule of any one of claims 1 to 3,
(b) the vector of claim 4,
(c) the peptide of claim 8, or
(d) the fusion molecule of any one of claims 9 to 11.

15. A method of detecting the internalization behaviour of a fusion molecule comprising the peptide of claim 8 or a fusion molecule according to any one of claims 9 to 11, comprising
(a) administering said fusion molecule to a cell
(b) detecting the internalization of the fusion molecule.

16. A method of treating and/or preventing a condition selected from cancer, enzyme deficiency diseases, infarcts, cerebral ischemia, diabetes, inflammatory diseases, infections such as bacterial, viral or fungal infections, autoimmune diseases such as systemic lupus erythematodes (SLE) or rheumatoid arthritis, diseases with amyloid-like fibrils such as Alzheimer's disease (AD) and Parkinson's disease (PD) or certain forms of myopathy comprising administering the composition of claim 12 or 13 to a subject in need thereof.

17. The peptide of claim 8, the fusion molecule of any one of claims 9 to 11 or the composition of claim 12 or 13 for the prevention and/or treatment of cancer, enzyme deficiency diseases, infarcts, cerebral ischemia, diabetes, inflammatory diseases, infections such as bacterial, viral or fungal infections, autoimmune diseases such as systemic lupus erythematodes (SLE) or rheumatoid arthritis, diseases with amyloid-like fibrils such as Alzheimer's disease (AD) and Parkinson's disease (PD) or certain forms of myopathy.

18. A kit comprising at least one of
(a) the nucleic acid molecule of any one of claims 1 to 3
(b) the vector of claim 4
(c) the host of claim 6
(d) the (poly)peptide of claim 8 or
(e) the fusion protein of any one of claims 9 to 11.
